# EUROPEAN PATENT APPLICATION

(11) **EP 4 840 541 A1**
(43) Date of publication of application: **18.11.2026**
(21) Application number: 25777837.3
(22) Date of filing: 12.03.2025
(51) Int. Cl.: B01J 23/89, B01J 31/04, B01J 37/02, B01J 37/16, C07B 61/00, C07C 67/055, C07C 69/15

(54) **METHOD FOR PRODUCING CATALYST FOR VINYL ACETATE PRODUCTION AND METHOD FOR PRODUCING VINYL ACETATE**

(30) Priority: 26.03.2024 JP 2024050557
(71) Applicant: Crasus Chemical Inc., Oita-shi, Oita 870-0189 (JP)
(72) Inventor: KITAGAWA, Kazuhiro, Oita-shi, Oita 870-0189 (JP); IWAMA, Yasuhiro, Oita-shi, Oita 870-0189 (JP); HOSOGI, Yasuhiro, Oita-shi, Oita 870-0189 (JP)
(74) Representative: Strehl & Partner mbB
(86) International application number: PCT/JP2025/009351
(87) International publication number: WO 2025/204921

(57) **Abstract**

The present invention provides a method for producing a catalyst that makes it possible to produce vinyl acetate with improved selectivity while ensuring high catalytic activity. Provided is a method for producing a catalyst that is for vinyl acetate production and that contains a carrier, palladium, gold, copper, and an acetate, said method comprising: step 1, which is for impregnating an alkaline solution into the carrier; step 2, which is for causing a solution containing a compound that includes palladium, a compound that includes gold, and a compound that includes copper to come into contact with and impregnate the carrier; step 5, which is for performing a liquid phase reduction treatment in the range of 0-15°C; and step 6, which is for causing the carrier to support the acetate.

## Description

### Technical Field

The present disclosure relates to a method for producing a catalyst for vinyl acetate production, the catalyst being used when vinyl acetate is produced using acetic acid, ethylene, and oxygen as raw materials, and a method for producing vinyl acetate using the catalyst.

### Background Art

Vinyl acetate is an important industrial material that is used in a wide range of fields such as, paints, adhesives, and fiber treatment agents, as a raw material for vinyl acetate resins, a raw material for polyvinyl alcohol, and a monomer for copolymerization with ethylene, styrene, acrylates, methacrylates, or the like.

As a catalyst for vinyl acetate production from acetic acid, ethylene, and oxygen as raw materials, a catalyst in which palladium, gold, and potassium acetate are supported on silica is widely used. In this reaction, the active sites are considered to be palladium, and gold is considered to play a role in suppressing the aggregation of palladium and, in addition, reducing the formation of carbon dioxide gas as a by-product, thereby improving the selectivity to vinyl acetate. For this effect of gold to be exhibited, gold atoms need to be present in close proximity to palladium. In PTL 1, by modifying the impregnation step into the carrier, palladium and gold are supported in a state where the supported locations of these metals are in close proximity to each other.

In the production of vinyl acetate, improving the selectivity to vinyl acetate is an important technical issue, and suppression of carbon dioxide gas formation is also desired from the viewpoint of environmental impact.

In PTL 2, the selectivity to vinyl acetate is improved by supporting copper in addition to palladium and gold.

In PTL 3, in a method for producing a catalyst containing palladium, gold, and copper, the selectivity to vinyl acetate is improved by supporting a palladium-containing compound, a gold-containing compound, and a copper-containing compound on a carrier in the same step.

### Citation List

### Patent Literature

PTL 1: JP 2008-080326 A
PTL 2: JP 2002-516749 T
PTL 3: WO 2022/113429

### Summary of Invention

### Technical Problem

In PTL 3, an alkali component is supported on a carrier, and subsequently, the carrier is impregnated with a solution containing a palladium-containing compound, a gold-containing compound, and a copper-containing compound in the same step, followed by hydrolysis of the compounds to support palladium hydroxide, gold hydroxide, and copper hydroxide on the carrier. Gold-containing compounds typified by chloroauric acid generally undergo hydrolysis slowly, and the rate at which they are supported on a carrier is low. Therefore, even when a palladium-containing compound and a gold-containing compound are brought into contact with a carrier on which an alkali component has been supported in the same step, due to the difference in hydrolysis rates between palladium and gold, gold atoms not in close proximity to palladium may be formed, or gold-containing compounds remaining in the solution may aggregate and become coarsened, thereby forming aggregates of gold particles. However, when a copper-containing compound is present during hydrolysis of a gold-containing compound, the copper-containing compound is rapidly hydrolyzed and supported on the carrier, and subsequently the gold-containing compound is adsorbed onto the hydrolysis product of the copper-containing compound supported on the carrier, whereby the hydrolysis rate of the gold-containing compound can be increased. Therefore, as compared with the case where no copper-containing compound is added in the same step, gold can be supported in a state of being in close proximity to palladium, and coarsening of gold particles in the solution can be suppressed; as a result, the selectivity to vinyl acetate can be improved. However, even in a catalyst produced by this method, aggregation of gold particles is observed; therefore, if aggregation of gold in the hydrolysis step and the reduction step of catalyst production can be further improved, further enhancement of catalyst performance can be expected.

The present disclosure provides a method for producing a catalyst that can produce vinyl acetate with improved selectivity while ensuring high catalytic activity.

### Solution to Problem

As a result of intensive studies to solve the above problem, the present inventors have found a method for producing a catalyst characterized in that compounds respectively containing palladium, gold, and copper are supported on a carrier while being reacted with an alkali component, followed by liquid-phase reduction treatment at a temperature in the range of from 0°C to 15°C, and have succeeded in producing vinyl acetate with improved selectivity.

That is, the present disclosure encompasses the following aspects [1] to [9].
[1] A method for producing a catalyst for vinyl acetate production, the catalyst comprising a carrier, palladium, gold, copper, and an acetate salt, the method comprising:
   step 1. impregnating the carrier with an alkaline solution;
   step 2. contact-impregnating the carrier with a solution comprising a palladium-containing compound, a gold-containing compound, and a copper-containing compound;
   step 5. performing a liquid-phase reduction treatment at a temperature in a range of 0°C to 15°C; and
   step 6. supporting an acetate salt on the carrier.
[2] The production method according to aspect [1], further comprising, between the step 2 and the step 5,
   step 3. separating the carrier from the solution in the step 2 when desired supported amounts of the catalyst components have been supported on the carrier.
[3] The production method according to aspect [2], further comprising, between the step 3 and the step 5,
   step 4. bringing the carrier into contact with water or an aqueous solution having a pH of 7.0 to 11.5.
[4] The production method according to any one of aspects [1] to [3], wherein a mass of supported metallic palladium per 1 kg of the carrier is from 5.00 g to 20.0 g.
[5] The production method according to any one of aspects [1] to [4], wherein a mass of supported metallic gold per 1 kg of the carrier is from 4.00 g to 20.0 g.
[6] The production method according to any one of aspects [1] to [5], wherein a mass of supported metallic copper per 1 kg of the carrier is from 0.25 g to 5.00 g.
[7] The production method according to any one of aspects [1] to [6], wherein a mass of supported acetate salt per 1 kg of the carrier is from 40 g to 125 g.
[8] The production method according to any one of aspects [1] to [7], wherein a mass ratio of palladium, gold, and copper is 100 : 70 to 130 : 8.3 to 9.5.
[9] A method for producing vinyl acetate, wherein a catalyst for vinyl acetate production obtained by the method according to any one of aspects [1] to [8] is used, and ethylene, oxygen, and acetic acid are used as raw materials.

### Advantageous Effects of Invention

According to the methods of the present disclosure, the selectivity to vinyl acetate can be significantly improved while ensuring high catalytic activity, as compared with known methods.

### Description of Embodiments

Hereinafter, embodiments of the present invention will be described, but the present invention is not limited only to these embodiments, and various variations can be made within the scope of the present invention. In the present disclosure, when "to" is used for a numerical range, the numerical values on both ends are an upper limit value and a lower limit value, respectively, and are included in the numerical range. When a plurality of upper limits or lower limits are listed, numerical ranges can be formed from all combinations of the upper limits and the lower limits. Similarly, when a plurality of numerical ranges are listed, upper limits and lower limits can be separately selected and combined from the numerical ranges to form separate numerical ranges.

### [Method for Producing Catalyst for Vinyl Acetate Production]

A method for producing a catalyst for vinyl acetate production of one embodiment includes the following steps, and these steps are preferably included in this order. Palladium, gold, and copper may be referred to as "catalyst components", and a palladium-containing compound, a gold-containing compound, and a copper-containing compound may be collectively, or individually for each metal, referred to as "raw material compounds". Steps 3 and 4 are optional steps that are preferably carried out.
Step 1. Impregnating a carrier with an alkaline solution
Step 2. Contact-impregnating the carrier with a solution (hereinafter referred to as "solution A") comprising a palladium-containing compound, a gold-containing compound, and a copper-containing compound, with palladium, gold, and copper serving as catalyst components
Step 3. Separating the carrier from the solution A when desired supported amounts of the respective catalyst components have been supported on the carrier (liquid draining step)
Step 4. Bringing the carrier into contact with water or an aqueous solution having a pH of 7.0 to 11.5 (buffer solution step)
Step 5. Performing a liquid-phase reduction treatment at a temperature in a range of 0°C to 15°C
Step 6. Supporting an acetate salt on the carrier

In one embodiment, the step 2 is carried out after the step 1, and the carrier is contact-impregnated with the solution A containing a palladium-containing compound, a gold-containing compound, and a copper-containing compound, thereby forming a catalyst precursor in which reaction products of these compounds with an alkaline compound are supported on the carrier. In the step 2, the amounts of the palladium-containing compound, the gold-containing compound, and the copper-containing compound in the solution A are preferably in excess relative to the desired supported amounts of the catalyst components.

The steps 1 to 6 are carried out in the above order; however, other steps may be included for purposes such as improving catalyst performance. For example, a step of washing the carrier with water after the step 5 may be included. The liquid-phase reduction treatment in the step 5 is a treatment for converting the reaction products of the raw material compounds with an alkaline compound into metallic palladium, metallic gold, and metallic copper, respectively, and therefore must be performed after the step 2 or the step 3. In addition, the step 5 and the step 6 may be interchanged in exceptional cases. Hereinafter, each step will be described in detail.

### <Step 1. Impregnating Carrier with Alkaline Solution>

In this step, the carrier is impregnated with an alkaline solution. This step can be carried out at room temperature. After completion of the impregnation operation, the carrier may be dried, or the process may proceed to the next step without performing operations such as drying.

The carrier is not particularly limited, and a porous material generally used as a catalyst carrier can be employed. The carrier is preferably silica, alumina, silica-alumina, diatomaceous earth, montmorillonite, or titania, and more preferably silica. When a material mainly composed of silica is used as the carrier, the silica content of the carrier is usually at least 50 mass% based on the mass of the carrier, and preferably at least 90 mass%.

The carrier preferably has a specific surface area, as measured by the BET method, of at least 1 m²/g, more preferably in the range of 10 to 1000 m²/g, and particularly preferably in the range of 100 to 500 m²/g. The bulk density of the carrier is preferably in the range of 50 to 1000 g/L, and particularly preferably in the range of 400 to 500 g/L. The water absorption rate of the carrier is preferably in the range of 0.05 to 3 g-water/g-carrier, and particularly preferably in the range of 0.1 to 2 g-water/g-carrier. Regarding the pore structure of the carrier, the average pore diameter is preferably in the range of 1 to 1000 nm, and particularly preferably in the range of 2 to 800 nm. When the average pore diameter is 1 nm or more, gas diffusion can be facilitated. On the other hand, when the average pore diameter is 1000 nm or less, a specific surface area of the carrier necessary for obtaining catalytic activity can be ensured.

For measuring a pore size distribution of a carrier, mercury intrusion porosimetry and the gas adsorption method (BJH method) are widely used. According to the IUPAC (International Union of Pure and Applied Chemistry) classification of pores, the mercury intrusion porosimetry can measure macropores of 50 nm or more and a part of mesopores of from 2 nm to less than 50 nm, whereas the gas adsorption method can measure mesopores and micropores of 2 nm or less. An appropriate measurement method can be selected according to the pore size.

In the present disclosure, the water absorption rate of the carrier refers to a value measured by the following measurement method.
1. About 5 g of the carrier is weighed using a balance (W1 g) and placed in a 100 mL beaker.
2. About 15 mL of pure water (ion-exchanged water) is added to the beaker so as to completely cover the carrier.
3. The mixture is allowed to stand for 30 minutes.
4. The contents of the beaker are poured onto a wire mesh having an opening of 1 mm, and pure water is drained off.
5. Water adhering to the surface of the carrier is removed by lightly pressing the carrier with a paper towel until the surface loses its gloss.
6. The total mass of the carrier and pure water is measured (W2 g).
7. The water absorption rate of the carrier is calculated from the following equation.$Water absorption rate \left(g-water/g-carrier\right) = \left(W2 - W1\right) / W 1$
Therefore, the water absorption amount (g) of the carrier is calculated by the water absorption rate (g-water/g-carrier) of the carrier × the mass (g) of the carrier used.

The shape of the carrier is not particularly limited. Specific examples include, but are not limited to, powder, spherical, and pellet forms. An optimal shape can be selected in accordance with the reaction mode, the reactor, and the like to be used.

There is also no particular limitation on the particle size of the carrier. When the carrier is spherical, the particle diameter is preferably in the range of 1 to 10 mm, and more preferably in the range of 3 to 8 mm. In a case where a gas-phase reaction is performed by packing a catalyst into a tubular reactor, when the particle diameter is 1 mm or more, an excessive increase in pressure loss during gas flow can be prevented, and gas circulation can be effectively performed. On the other hand, when the particle diameter is 10 mm or less, diffusion of the raw material gas into the interior of the catalyst is facilitated, thereby allowing the catalytic reaction to proceed effectively. Furthermore, since the number of catalyst particles packed into the tubular reactor does not decrease excessively, a sufficient total surface area of the catalyst particles can be secured to ensure appropriate amounts of metal components (such as palladium, gold, and copper) dispersed on the surface of the carrier for the reaction.

The alkaline solution may be a solution of any alkaline compound. Examples of the alkaline compound include hydroxides of alkali metals or alkaline earth metals, bicarbonates of alkali metals or alkaline earth metals, carbonates of alkali metals or alkaline earth metals, and silicates of alkali metals or alkaline earth metals. As the alkali metal, lithium, sodium, or potassium can be used. As the alkaline earth metal, barium or strontium can be used. As the alkaline compound, sodium metasilicate, potassium metasilicate, sodium hydroxide, potassium hydroxide, barium hydroxide, or strontium hydroxide is preferably used.

The solvent of the alkaline solution is not particularly limited, and examples thereof include water, methanol, and ethanol, with water being preferred.

The alkaline compound is used in excess relative to the total amount of palladium, gold, and copper described below. For example, the product of the molar amount of the alkaline compound and the valence of the alkaline compound is preferably more than 1.1 times and 3.0 times or less, and more preferably more than 1.5 times and 2.0 times or less, the total of the product of the molar amount of palladium element and the valence of palladium in the palladium-containing compound, the product of the molar amount of gold element and the valence of gold in the gold-containing compound, and the product of the molar amount of copper element and the valence of copper in the copper-containing compound.

There is no particular limitation on the method for impregnating the carrier with the alkaline solution. For example, (I) a method in which the carrier is immersed in a large amount of an alkaline solution for a certain period and then the carrier impregnated with an amount of the alkaline solution corresponding to its water absorption amount is taken out, and (II) a method in which an alkaline compound is dissolved in a solvent and the carrier is impregnated with a solution adjusted to a volume corresponding to the water absorption amount of the carrier, may be employed. From the viewpoint of wastewater treatment, the method (II) is preferred.

The carrier is preferably impregnated with the alkaline solution in an amount corresponding to from 0.9 times to 1.0 times the water absorption amount of the carrier on a mass basis, and more preferably impregnated with the alkaline solution in an amount corresponding to from 0.95 times to 1.0 times the water absorption amount of the carrier on a mass basis. When the amount of the alkaline solution is at least 0.9 times the water absorption amount of the carrier on a mass basis, uneven impregnation of the alkaline solution is less likely to occur. When the amount of the alkaline solution is at most 1.0 times the water absorption amount of the carrier on a mass basis, the entire amount of the alkaline solution can be reliably absorbed into the carrier. In the present disclosure, the water absorption amount of the carrier is a value measured using pure water and, strictly speaking, differs from the value for the alkaline solution; however, it is used as it is for convenience.

### <Step 2. Contact-impregnating Carrier with Solution A>

In this step, the carrier impregnated with the alkaline solution is contact-impregnated with a solution A. The solution A is a solution containing a palladium-containing compound, a gold-containing compound, and a copper-containing compound. The solution A may contain other components, as necessary.

The amounts of the raw material compounds (the palladium-containing compound, the gold-containing compound, and the copper-containing compound) contained in the solution A may be equal to the desired catalyst composition, that is, the supported amounts of the catalyst components (metallic palladium, metallic gold, and metallic copper) in the finally obtained catalyst; however, it is preferable to charge the raw material compounds (the palladium-containing compound, the gold-containing compound, and the copper-containing compound) in amounts in excess relative to the amounts of the catalyst components in the finally obtained catalyst. That is, the raw material compound, in an amount obtained by multiplying the amount of the raw material compound corresponding to the desired supported amount of the catalyst component by each excess ratio, is dissolved such that each of the palladium-containing compound, the gold-containing compound, and the copper-containing compound is present in excess relative to the desired supported amount of the catalyst component. The concentrations of the raw material compounds of the catalyst components (palladium, gold, and copper) in the solution A can be calculated from the amounts of the raw material compounds calculated as described above and the solution volume. In actual operation, it is sufficient to weigh out the required amount (g) of the raw material compound and dissolve it in a solvent so as to obtain a preferred solution volume.

The excess ratio of the charged amount of the raw material compound for each catalyst component in the solution A is calculated by the following equation. 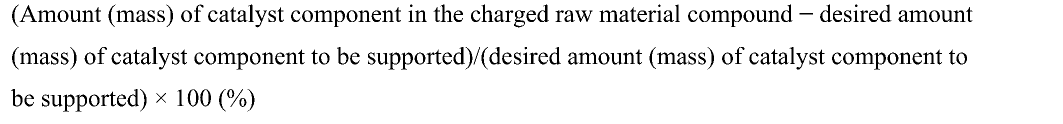 (Amount (mass) of catalyst component in the charged raw material compound - desired amount (mass) of catalyst component to be supported)/(desired amount (mass) of catalyst component to be supported) × 100 (%)

When the catalyst component is gold, the amount (g) of a gold-containing compound (e.g., chloroauric acid) corresponding to the amount (g) to be supported per 1 kg of the carrier is calculated, and an amount (g) of the gold-containing compound obtained by multiplying this by the amount of the carrier (kg) and (excess ratio of the gold-containing compound + 100)/100 is dissolved in the solvent of the solution A. Specifically, when the amount (g) of gold to be supported per 1kg of the carrier is 10 g, the amount of chloroauric acid (HAuCl₄) containing 10 g of gold atoms is 17.26 g. When the excess ratio is 80%, the charged amount of chloroauric acid per 1 kg of the carrier is 31.068 g (= 17.26 × ((80 + 100)/100). An amount of chloroauric acid obtained by multiplying this value by the amount (kg) of the carrier to be used is dissolved in the solvent of the solution A. The solution A is prepared in the same manner for palladium and copper.

Regarding the excess ratio of the charged amount of the raw material compound of each catalyst component in the solution A, the excess ratio of the charged amount of the palladium-containing compound is preferably from 2% to 50%, more preferably from 5% to 40%, and still more preferably from 10% to 20%. The excess ratio of the charged amount of the gold-containing compound is preferably from 2% to 150%, more preferably from 10% to 100%, and still more preferably from 25% to 90%. The excess ratio of the charged amount of the copper-containing compound is preferably from 2% to 50%, more preferably from 5% to 40%, and still more preferably from 8% to 15%.

By using the solution A containing the raw material compounds in amounts in excess relative to the desired supported amounts of the catalyst components, the contact time between the carrier and the solution A required to obtain the desired supported amounts of the catalyst components can be shortened. This suppresses coarsening of gold particles during the processes of dissolution and reprecipitation. When the solution A containing the raw material compounds in amounts in excess relative to the desired supported amounts of the catalyst components is used, it is preferable to carry out the step 3 (liquid draining step) when the desired supported amounts of the catalyst components have been supported on the carrier. The timing for proceeding to the step 3 is determined based on preliminary experiments. The details thereof will be described later.

As the palladium-containing compound in the solution A, a palladium precursor that can be converted to metallic palladium can be used. Examples of the palladium precursor that can be converted to metallic palladium include palladium chloride, palladium nitrate, palladium sulfate, sodium chloropalladate, potassium chloropalladate, barium chloropalladate, and palladium acetate, with sodium chloropalladate being preferred.

As the gold-containing compound in the solution A, a gold precursor that can be converted to metallic gold can be used. Examples of the gold precursor include chloroauric acid, sodium chloroaurate, and potassium chloroaurate, with chloroauric acid being preferred.

As the copper-containing compound in the solution A, a copper precursor that can be converted to metallic copper can be used. Examples of the copper precursor that can be converted to metallic copper include copper chloride, copper acetate, and copper nitrate, with copper chloride being preferred.

Examples of the solvent of the solution A include water, alcohols, and organic acids. Water is preferred because it does not damage the carrier and does not react with the compounds contained in the solution A.

The amount of the solution A is preferably from 1.0 to 10.0 times the water absorption amount of the carrier on a mass basis, more preferably from 2.0 to 8.0 times, and particularly preferably from 2.0 to 5.0 times.

By bringing the solution A into contact with the carrier impregnated with the alkaline solution to impregnate the carrier, the raw metal compounds can be converted into water-insoluble substances, thereby forming a shell-type catalyst precursor in which metal components, such as palladium, gold, and copper, are supported in a state of being localized at the surface portion of the carrier.

The contact temperature is not particularly limited, but is preferably from 10 to 80°C, and more preferably from 20 to 60°C. When the contact temperature is 10°C or higher, the conversion reaction can be sufficiently promoted. When the contact temperature is 80°C or lower, aggregation of gold, palladium, and copper can be suppressed.

The contact time is set to a time required to reach the desired supported amount, after confirming, through repeated preliminary experiments, the time-dependent change in the loading of the catalyst components at a predetermined contact temperature. Since the contact time cannot be varied for each catalyst component, the excess ratios of the charged amounts of the raw material compounds are adjusted so that the desired supported amounts are obtained at the contact time. The contact time is preferably from 0.5 to 100 hours, and more preferably from 1 to 20 hours. When the contact time is 100 hours or less, deterioration of the carrier can be suppressed.

### <Step 3. Liquid draining step>

In this step, only the carrier is taken out from the mixture of the carrier and the solution obtained in step 2. Gold-containing compounds, such as chloroauric acid, have a significantly lower hydrolysis rate than palladium-containing compounds and copper-containing compounds; therefore, when the carrier is immersed in the solution A for a long time, compositional non-uniformity is likely to occur within the carrier. In addition, when the carrier is immersed in the solution A for a long time, coarsening of gold particles is likely to occur during the processes of dissolution and reprecipitation. In contrast, by separating the carrier from the solution A when the desired amounts of the catalyst components have been supported on the carrier, occurrence of compositional non-uniformity and coarsening of gold particles can be suppressed. There is no particular limitation on the method for separating the carrier and the solution, and it can be appropriately selected from known methods in consideration of the shape, form, and the like of the catalyst. For example, a filtration method, a centrifuge method, or a sedimentation method can be used, with a filtration method being preferred. Examples of the filtration method include a method in which the mixture is passed through a mesh having openings smaller than the size of the carrier. When the carrier size is from 3 to 8 mm, the mixture may be passed through a mesh having, for example, an opening size of 1 mm. It is preferable that, by solid-liquid separation, the solution be removed to a level of not more than 1.2 times the water absorption rate of the carrier.

### <Step 4. Buffer Solution Step>

In this step, water or an aqueous solution having a pH of 7.0 to 11.5 (hereinafter, collectively referred to as "solution B") is brought into contact with the carrier that has been impregnated with the solution A and separated from the solution. Although this is an assumption, it is considered that, by the operation of the step 4, the state of gold can be brought closer to Au(OH)₃ without Cl⁻ coordination, and an effect of suppressing aggregation of gold in the subsequent step 5 (liquid-phase reduction treatment step) is expected. The solution B may be gently stirred; however, it is preferably allowed to stand in order to prevent breakage of the carrier.

Gold-containing compounds typified by chloroauric acid form AuCl_{3 - n}(OH)ₙ and Au(OH)₃ by reacting with an alkali component. In the liquid-phase reduction treatment step of the step 5, when gold is reduced in a state where Cl⁻ is coordinated to gold, an increase in the particle size of gold and aggregation of gold particles are likely to occur. The higher the pH of the solution, the more the substitution from Cl⁻ to OH is promoted. On the other hand, when the pH of the solution becomes too high, gold is redissolved as [Au(OH)₄]⁻. In addition, when the solution temperature is high, an increase in the particle size of gold and aggregation of gold particles are likely to proceed.

By carrying out the buffer solution step, the pH of the solution after reaction with the alkali component can be adjusted to a range in which the state of gold is brought closer to a state without Cl⁻ coordination, that is, Au(OH)₃, while preventing redissolution of gold; and, by performing reduction at a low temperature, aggregation of gold particles in the liquid-phase reduction treatment step can be suppressed, thereby enabling production of a catalyst for vinyl acetate production having a palladium-gold alloy state suitable for the vinyl acetate synthesis reaction.

The solution B may be any water or aqueous solution having a pH of 7.0 to 11.5 at the operating temperature in the step 4 (e.g., room temperature (20°C)). The pH is the pH of the solution B before being brought into contact with the carrier. The pH is preferably from 8.0 to 11.0. During the step 4, the pH may deviate from the above range; however, it is preferable to maintain the pH within the above range. In the present disclosure, the pH is a value measured at the temperature in the step 4 using a pH meter (benchtop pH meter, HORIBA Advanced Techno Co., Ltd.). The solution B may be water alone or an alkaline aqueous solution. Other components may be dissolved in the solution B, as necessary.

When the solution B is an alkaline aqueous solution, the solution may be an aqueous solution of any alkaline compound. Examples of the alkaline compound include hydroxides of alkali metals or alkaline earth metals, bicarbonates of alkali metals or alkaline earth metals, carbonates of alkali metals or alkaline earth metals, and silicates of alkali metals or alkaline earth metals. As the alkali metal, lithium, sodium, or potassium can be used. As the alkaline earth metal, barium or strontium can be used. As the alkaline compound, sodium metasilicate, potassium metasilicate, sodium hydroxide, potassium hydroxide, barium hydroxide, or strontium hydroxide is preferably used.

As the solution B, a pH buffer solution is preferably used because the change in pH during the step 4 becomes small. The pH of the pH buffer solution before being brought into contact with the carrier is preferably from 7.0 to 11.5, and more preferably from 8.0 to 11.0. Use of a pH buffer solution prevents the pH of the solution B from becoming too high, thereby preventing redissolution of gold. Examples of the pH buffer solution include a phosphate (sodium) buffer solution (pH from 5.8 to 7.8), a citrate (sodium) buffer solution (pH from 6.0 to 8.0), a phosphate-buffered saline (pH from 6.4 to 8.4), an ethylenediaminetetraacetate (sodium) buffer solution (pH from 7.0 to 9.0), a Tris-HCl buffer solution (pH from 7.0 to 9.0), a Tris-EDTA buffer solution (pH from 7.0 to 9.0), an acetate-ethanolamine buffer solution (pH from 8.6 to 10.6), a borate (potassium) buffer solution (e.g., pH from 8.1 to 10.1), a borate (sodium) buffer solution (e.g., pH 9.1), and a carbonate-bicarbonate buffer solution (e.g., pH 10.0), with a borate (potassium) buffer solution, a borate (sodium) buffer solution, or a carbonate-bicarbonate buffer solution being preferred, and a carbonate-bicarbonate buffer solution being more preferred. In the above buffer solutions, (sodium) or (potassium) indicates that the counterion is sodium or potassium, respectively.

The amount of the solution B is preferably from 1.0 to 10.0 times the water absorption amount of the carrier on a mass basis, more preferably from 2.0 to 8.0 times, and particularly preferably from 2.0 to 5.0 times.

The temperature of the solution B in the step 4 is not particularly limited, but is preferably from 10 to 80°C, and more preferably from 20 to 60°C. By setting the contact temperature to 10°C or higher, the conversion reaction can be sufficiently promoted. By setting the contact temperature to 80°C or lower, aggregation of gold, palladium, and copper can be suppressed.

The contact time with the solution B is preferably from 0.1 hours to 48 hours, and more preferably from 1.0 hour to 24 hours.

### <Step 5. Liquid-Phase Reduction Treatment Step>

It is desirable to subject the carrier on which reaction products of the raw material compounds and the alkaline compound are supported to a reduction treatment, thereby converting the above reaction products into metallic palladium, metallic gold, and metallic copper. In the present disclosure, "metallic copper", "metallic palladium", and "metallic gold" refer to metal species having a valence of zero. The reduction treatment is performed in a liquid-phase. It is not necessary that all of the palladium, gold, and copper be reduced to the metallic state, that is, the zero-valent state by the reduction treatment.

The liquid-phase reduction can be performed in either a non-aqueous system using alcohols or hydrocarbons, or an aqueous system. As the reducing agent, for example, a carboxylic acid and a salt thereof, an aldehyde, hydrogen peroxide, a saccharide, polyhydric phenol, a boron compound, an amine, or hydrazine can be used. Examples of the carboxylic acid and salt thereof include oxalic acid, potassium oxalate, formic acid, potassium formate, potassium citrate, and ammonium citrate. Examples of the aldehyde include formaldehyde and acetaldehyde. Examples of the saccharide include glucose. Examples of the polyhydric phenol include hydroquinone. Examples of the boron compound include diborane and sodium borohydride. As the reducing agent, hydrazine, formaldehyde, acetaldehyde, hydroquinone, sodium borohydride, and potassium citrate are preferred, with hydrazine being particularly preferred.

In the liquid-phase reduction treatment, the liquid-phase temperature is set in the range of 0 to 15°C. The temperature is preferably in the range of 0 to 13°C, more preferably in the range of 0 to 10°C, and still more preferably in the range of 3 to 7°C. When the liquid-phase temperature, that is, the liquid-phase reduction treatment temperature, is in the range of 0 to 15°C, the selectivity to vinyl acetate can be increased. The reduction time is preferably in the range of 0.5 to 24 hours, and more preferably in the range of 1 to 10 hours. When the reduction time is 0.5 hours or more, the reduction can be sufficiently promoted. On the other hand, when the reduction time is 24 hours or less, aggregation of palladium, gold, and copper can be suppressed.

The reduced carrier is washed with pure water or the like, as necessary. The washing may be performed continuously or in batches. The washing temperature is preferably in the range of 5 to 200°C, and more preferably in the range of 15 to 80°C. There is no particular limitation on the washing time, and conditions sufficient for the purpose of removing undesirable residual impurities may be selected. Examples of the undesirable impurities include chlorine-containing compounds and chloride ions. After the washing, the carrier may be dried by heating, as necessary.

### <Step 6. Supporting Acetate Salt on Carrier>

The acetate salt can be supported by impregnating the carrier with a solution containing the required amount of the acetate salt, followed by drying. The amount of the acetate solution used is preferably from 0.9 to 1 times the water absorption amount of the carrier on a mass basis. The acetate salt is typically supported after the reduction treatment, but may also be supported before the reduction treatment.

The acetate salt is preferably at least one compound selected from the group consisting of alkali metal acetates and alkaline earth metal acetates, and more preferably an alkali metal acetate. Examples of the alkali metal acetate include acetates of lithium, sodium, and potassium. As the acetate salt, sodium acetate and potassium acetate are preferred, with potassium acetate being particularly preferred.

The supported amount of the acetate salt is preferably such that the mass of the supported acetate salt per 1 kg of the carrier is from 40 g to 125 g.

### [Catalyst for Vinyl Acetate Production]

The mass of the supported metallic palladium per 1 kg of the carrier is preferably from 5.00 g to 20.0 g, more preferably from 8.0 g to 17.0 g, and still more preferably from 10.0 g to 15.0 g.

The mass of the supported metallic gold per 1 kg of the carrier is preferably from 4.00 g to 20.0 g, more preferably from 6.00 g to 18.0 g, and still more preferably from 10.0 g to 17.0 g.

The mass of the supported metallic copper per 1kg of the carrier is preferably from 0.25 g to 5.00 g, more preferably from 0.60 g to 2.50 g, and still more preferably from 0.80 g to 1.5 g.

The mass of the supported acetate salt per 1 kg of the carrier is preferably from 40 g to 125 g, and more preferably from 50 g to 88 g.

The mass ratio of palladium, gold, and copper is preferably palladium : gold : copper = 100 : 70 to 130 : 8.3 to 9.5.

The supported amounts of the metal elements and the acetate salt are measured by the methods described in Examples.

The catalyst for vinyl acetate production obtained by the method of the present disclosure has an eggshell structure in which most of the palladium, gold, and copper are supported on the surface portion of the carrier. The thickness of the shell portion varies depending on the types of the carrier, the alkaline solution, the solution containing the compounds containing the catalytic metal elements, and the like used. When spherical silica having a diameter of 5 mm is used as the carrier, the shell portion preferably has a thickness of from 0.05 to 0.5 mm, and more preferably from 0.1 to 0.3 mm. When the thickness of the shell portion is 0.05 mm or more, catalytic activity can be maintained even if the surface portion of the carrier peels off during the reaction. When the thickness of the shell portion is 0.5 mm or less, the catalyst concentration in the surface portion of the carrier can be sufficiently increased, and the advantages of shell-type supporting can be economically achieved. The acetate salt does not need to be supported in a shell form, and may be uniformly present throughout the catalyst.

### [Specific Example of Catalyst Production]

A preferred specific example of the catalyst production is shown below.
1. The carrier is impregnated with an alkaline solution in an amount corresponding to the water absorption amount of the carrier.
2. A catalyst precursor on which desired amounts of catalyst components are supported is formed by immersing the carrier in a solution A for a predetermined period of time to contact-impregnate the carrier with the solution A, the solution A being obtained by adjusting the volume, with pure water, of excessive amounts of a palladium-containing compound, a gold-containing compound, and a copper-containing compound relative to the desired supported amounts of the catalyst components to 2 times the water absorption amount of the carrier on a mass basis.
3. The catalyst precursor that has been contact-impregnated is separated from the solution obtained in 2.
4. The catalyst precursor obtained in 3 is allowed to stand in water or an aqueous solution having a pH of 7.0 to 11.5.
5. The catalyst precursor obtained in 4 is brought into contact with a reducing agent to perform a liquid-phase reduction treatment.
6. The carrier after reduction is washed with pure water.
7. The washed carrier is dried.
8. A predetermined amount of the acetate salt is supported on the carrier.
9. The carrier on which the catalyst components are supported is dried.

### [Production of Vinyl Acetate]

Hereinafter, a method for producing vinyl acetate using the catalyst for vinyl acetate production produced by the method of the present disclosure will be described. The reaction for producing vinyl acetate is preferably performed in a gas phase using acetic acid, ethylene, and oxygen as reaction raw materials. The gas-phase reaction may be in any commonly known form, but is preferably a fixed-bed flow reaction.

The reaction formula is as follows.

CH₂ = CH₂ + CH₃COOH + 1/2O₂→CH₂ = CHOCOCH₃ + H₂O

The ratio of acetic acid, ethylene, and oxygen in the raw material gas is preferably a molar ratio of acetic acid : ethylene : oxygen = 1 : 0.08 to 16 : 0.01 to 4, and more preferably a molar ratio of acetic acid : ethylene : oxygen = 1 : 0.2 to 9 : 0.07 to 2.

The raw material gas contains ethylene, acetic acid (gas), and oxygen gas, and may further contain, as a diluent, nitrogen gas, carbon dioxide, a noble gas, or the like, as necessary. When ethylene, acetic acid, and oxygen are defined as the reaction raw materials, the ratio of the reaction raw materials to the diluent is preferably a molar ratio of reaction raw materials : diluent = 1 : 0.05 to 9, and more preferably a molar ratio of reaction raw materials : diluent = 1 : 0.1 to 3.

When the reaction is performed by a fixed-bed flow reaction, the raw material gas is preferably supplied to the reactor at a space velocity (SV) of 10 to 15000 hr⁻¹, and more preferably supplied to the reactor at 300 to 12000 hr⁻¹ in the standard state. When the space velocity is 10 hr⁻¹ or more, the reaction heat can be appropriately removed. On the other hand, when the space velocity is 15000 hr⁻¹ or less, equipment such as a compressor can be made to have a practical size.

It is preferable to add water to the raw material gas as steam in an amount of 0.5 to 20 mol%, and more preferably 1 to 18 mol%. Without being bound by any theory, it is considered that the presence of water in the reaction system suppresses elution of the acetate salt from the catalyst. Even if water is added in an amount exceeding 20 mol%, the above effect is not improved, and hydrolysis of vinyl acetate may proceed. Therefore, it is not preferable for a large amount of water to be present in the raw material gas.

There is no particular limitation on the material of the reactor, but it is preferably a material having corrosion resistance.

The reaction temperature is preferably in the range of 100 to 300°C, and more preferably in the range of 120 to 250°C. When the reaction temperature is 100°C or higher, an appropriate reaction rate can be maintained. When the reaction temperature is 300°C or lower, the reaction heat can be easily removed.

The reaction pressure is preferably in the range of 0 to 3 MPaG (gauge pressure), and more preferably in the range of 0.1 to 1.5 MPaG. When the reaction pressure is 0 MPaG or higher, an appropriate reaction rate can be maintained. When the reaction pressure is 3 MPaG or lower, it is unnecessary to provide equipment, such as reaction tubes, with high pressure resistance, and equipment costs can be suppressed.

It is preferable to use high-purity ethylene as the reaction raw material, but lower saturated hydrocarbons, such as methane, ethane, and propane, may be mixed therein.

The ethylene and acetic acid as the reaction raw materials may each be produced from fossil-based raw materials or produced from biomass-based raw materials, or may be mixtures thereof. The concentrations of ¹⁴C atoms in ethylene, acetic acid, and vinyl acetate may each be from 1 × 10⁻¹⁴ to 2 × 10⁻¹² relative to the total number of carbon atoms.

There is also no particular limitation on the oxygen gas. The oxygen gas may be supplied in a form diluted with an inert gas such as nitrogen gas or carbon dioxide gas, for example, in the form of air. However, when the reaction gas is circulated, it is generally advantageous to use high-concentration oxygen gas, preferably oxygen gas having a purity of 99% or more.

### Examples

Hereinafter, the present invention will be further described by way of Examples; however, the present invention is not limited only to these Examples.

### (Example 1) Preparation of Catalyst A

Using, as a carrier, a commercially available spherical silica carrier (particle diameter: 5 mm, BET specific surface area: 176 m²/g, bulk density: 470 g/L, water absorption rate: 0.85 g-water/g-carrier, average pore size: 17.2 nm; hereinafter simply referred to as "support"), a catalyst was prepared according to the following procedure.

Step 1. 23.5 g of the carrier (water absorption amount: 20.0 g) was impregnated with an aqueous solution containing 4.9 g of Na₂SiO₃·9H₂O in an amount corresponding to the water absorption amount of the carrier (1.1 times on a mass basis). The container containing the carrier and the aqueous solution was shaken to completely impregnate the carrier with the solution, and then allowed to stand in a sealed container for 3 hours. The water absorption amount was calculated from the carrier amount of 23.5 g and the water absorption rate of 0.85 g-water/g-carrier (the same applies to the following Examples and Comparative Examples).

Step 2. The carrier obtained in the step 1 was immersed in an aqueous solution, in an amount equal to 2 times the water absorption amount of the carrier on a mass basis, containing 1.06 g of Na₂PdCl₄, 0.80 g of HAuCl₄, and 0.074 g of copper chloride dihydrate, and allowed to stand at room temperature (25°C) for 2 hours, thereby obtaining a catalyst precursor on which desired amounts of catalyst components are supported. The standing time was determined from the relationship between the amounts of supported catalyst components and time, which had been determined in advance by preliminary experiments, based on the time required to achieve the desired supported amounts. The desired supported amounts of the catalyst components were 13.8 g of palladium, 12.5 g of gold, and 1.15 g of copper per 1 kg of the carrier. The excess ratios of the charged amounts of the raw material compounds relative thereto were set such that the palladium-containing compound was 18.3%, the gold-containing compound was 56.8%, and the copper-containing compound was 2.0%.

Step 3. The catalyst precursor was separated from the dispersion obtained in the step 2 using a metal mesh having an opening size of 1 mm, followed by liquid draining.

Step 4. The catalyst precursor obtained in the step 3 was immersed at 25°C in ion-exchanged water (pH of 7.0) in an amount equal to 4 times the water absorption amount of the carrier on a mass basis, and allowed to stand at room temperature (25°C) for 18 hours.

Step 5. The catalyst precursor was separated from the dispersion obtained in the step 4 using the same metal mesh as described above, followed by liquid draining. The separated catalyst precursor was immersed in an aqueous solution, in an amount equal to 2 times the water absorption amount of the carrier on a mass basis, containing 5.9 g of a 52 mass% hydrazine hydrate aqueous solution, and allowed to stand in an ice bath (5°C) for 4 hours to perform a liquid-phase reduction treatment. Next, the palladium/gold/copper/support composition was washed with deionized water, and the washing was continued until no chloride ions remained in the water after washing. The washed palladium/gold/copper/support composition was dried with hot air at 110°C for 4 hours.

Step 6. The palladium/gold/copper/support composition obtained in the step 5 was impregnated at room temperature (25°C) with an aqueous solution, in an amount corresponding to 0.9 times the water absorption amount of the carrier on a mass basis, containing 1.7 g of potassium acetate, and then dried with hot air at 110°C for 4 hours to obtain a catalyst A.

### (Example 2) Preparation of Catalyst B

Using the same spherical silica carrier as in Example 1, a catalyst was prepared according to the following procedure.

Step 1. 23.5 g of the carrier (water absorption amount: 20.0 g) was impregnated with an aqueous solution containing 4.4 g of Na₂SiO₃·9H₂O in an amount corresponding to the water absorption amount of the carrier (1.1 times on a mass basis). The container containing the carrier and the aqueous solution was shaken to completely impregnate the carrier with the solution, and then allowed to stand in a sealed container for 3 hours.

Step 2. The carrier obtained in the step 1 was immersed in an aqueous solution, in an amount equal to 2 times the water absorption amount of the carrier on a mass basis, containing 1.06 g of Na₂PdCl₄, 0.80 g of HAuCl₄, and 0.074 g of copper chloride dihydrate, and allowed to stand at room temperature (25°C) for 20 hours, thereby obtaining a catalyst precursor on which desired amounts of catalyst components are supported. The standing time was determined from the relationship between the amounts of supported catalyst components and time, which had been determined in advance by preliminary experiments, based on the time required to achieve the desired supported amounts. The desired supported amounts of the catalyst components were 13.4 g of palladium, 16.8 g of gold, and 1.13 g of copper per 1 kg of the carrier. The excess ratios of the charged amounts of the raw material compounds relative thereto were set such that the palladium-containing compound was 22.0%, the gold-containing compound was 17.0%, and the copper-containing compound was 4.0%.

Step 3. The catalyst precursor was separated from the dispersion obtained in the step 2 using a metal mesh having an opening size of 1 mm, followed by liquid draining.

### Step 5.

The separated catalyst precursor was immersed in an aqueous solution, in an amount equal to 2 times the water absorption amount of the carrier on a mass basis, containing 5.9 g of a 52 mass% hydrazine hydrate aqueous solution, and allowed to stand in an ice bath (5°C) for 4 hours to perform a liquid-phase reduction treatment. Next, the palladium/gold/copper/support composition was washed with deionized water, and the washing was continued until no chloride ions remained in the water after washing. The washed palladium/gold/copper/support composition was dried with hot air at 110°C for 4 hours.

Step 6. The palladium/gold/copper/support composition obtained in the step 5 was impregnated at room temperature (25°C) with an aqueous solution, in an amount corresponding to 0.9 times the water absorption amount of the carrier on a mass basis, containing 1.7 g of potassium acetate, and then dried with hot air at 110°C for 4 hours to obtain a catalyst B.

### (Example 3) Preparation of Catalyst C

Using the same spherical silica carrier as in Example 1, a catalyst was prepared according to the following procedure.

Step 1. 23.5 g of the carrier (water absorption amount: 20.0 g) was impregnated with an aqueous solution containing 4.9 g of Na₂SiO₃·9H₂O in an amount corresponding to the water absorption amount of the carrier (1.1 times on a mass basis). The container containing the carrier and the aqueous solution was shaken to completely impregnate the carrier with the solution, and then allowed to stand in a sealed container for 3 hours.

Step 2. The carrier obtained in the step 1 was immersed in an aqueous solution, in an amount equal to 2 times the water absorption amount of the carrier on a mass basis, containing 0.89 g of Na₂PdCl₄, 0.62 g of HAuCl₄, and 0.091 g of copper chloride dihydrate, and allowed to stand at room temperature (25°C) for 20 hours, thereby obtaining a catalyst precursor on which desired amounts of catalyst components are supported. The standing time was determined from the relationship between the amounts of supported catalyst components and time, which had been determined in advance by preliminary experiments, based on the time required to achieve the desired supported amounts. The desired supported amounts of the catalyst components were 12.9 g of palladium, 12.0 g of gold, and 1.17 g of copper per 1 kg of the carrier. The excess ratios of the charged amounts of the raw material compounds relative thereto were set such that the palladium-containing compound was 6.8%, the gold-containing compound was 28.1%, and the copper-containing compound was 23.6%.

Step 3. The catalyst precursor was separated from the dispersion obtained in the step 2 using a metal mesh having an opening size of 1 mm, followed by liquid draining.

Step 4. The catalyst precursor obtained in the step 3 was immersed at 25°C in a pH buffer solution (pH = 10.0), in an amount equal to 4 times the water absorption amount of the carrier on a mass basis, containing 0.86 g of NaHCO₃ and 0.68 g of Na₂CO₃, and allowed to stand at 25°C for 3 hours.

Step 5. The catalyst precursor was separated from the dispersion obtained in the step 4 using the same metal mesh as described above, followed by liquid draining. The separated catalyst precursor was immersed in an aqueous solution, in an amount equal to 2 times the water absorption amount of the carrier on a mass basis, containing 4.8 g of a 52 mass% hydrazine hydrate aqueous solution, and allowed to stand at 5°C for 4 hours to perform a liquid-phase reduction treatment. Next, the palladium/gold/copper/support composition was washed with deionized water, and the washing was continued until no chloride ions remained in the water after washing. The washed palladium/gold/copper/support composition was dried with hot air at 110°C for 4 hours.

Step 6. The palladium/gold/copper/support composition obtained in the step 5 was impregnated at 25°C with an aqueous solution, in an amount corresponding to 0.9 times the water absorption amount of the carrier on a mass basis, containing 1.7 g of potassium acetate, and then dried with hot air at 110°C for 4 hours to obtain a catalyst C.

### (Example 4) Preparation of Catalyst D

A catalyst D was obtained by performing the same operation as in Example 3, except that the temperature of the liquid-phase reduction treatment in the step 5 of Example 3 was changed to 15°C.

### (Comparative Example 1) Preparation of Catalyst E

A catalyst E was obtained by performing the same operation as in Example 1, except that the temperature of the liquid-phase reduction treatment in the step 5 of Example 1 was changed to 25°C.

### (Comparative Example 2) Preparation of Catalyst F

Using the same spherical silica carrier as in Example 1, a catalyst was prepared according to the following procedure.

Step 1. 23.5 g of the carrier (water absorption amount: 20.0 g) was impregnated with an aqueous solution containing 7.5 g of Na₂SiO₃·9H₂O in an amount corresponding to the water absorption amount of the carrier (2.9 times on a mass basis). The container containing the carrier and the aqueous solution was shaken to completely impregnate the carrier with the solution, followed by air drying for 5 minutes. The water absorption amount was calculated from the carrier amount of 23.5 g and the water absorption rate of 0.85 g-water/g-carrier.

Step 2. The carrier obtained in the step 1 was immersed in an aqueous solution, in an amount equal to 2 times the water absorption amount of the carrier on a mass basis, containing 0.87 g of Na₂PdCl₄, 0.38 g of HAuCl₄, and 0.04 g of copper chloride dihydrate, and allowed to stand at room temperature for 20 hours.

Step 5. To the dispersion obtained in the step 2, 1.9 g of a 52 mass% hydrazine hydrate aqueous solution was added, followed by gentle mixing, and the mixture was allowed to stand at 25°C for 4 hours to perform a liquid-phase reduction treatment. Next, the palladium/gold/copper/support composition obtained in the previous step was washed with deionized water, and the washing was continued until no chloride ions remained in the water after washing. The washed palladium/gold/copper/support composition was dried with hot air at 110°C for 4 hours.

Step 6. The palladium/gold/copper/support composition obtained in the step 5 was impregnated at room temperature (25°C) with an aqueous solution, in an amount corresponding to 0.9 times the water absorption amount of the carrier on a mass basis, containing 1.7 g of potassium acetate, and then dried with hot air at 110°C for 4 hours to obtain a catalyst F.

### [Evaluation of Catalyst]

### <Measurement of Supported Amounts of Metals (Palladium, Gold, and Copper) and Potassium Acetate>

A 3 g sample of the supported catalyst was pulverized and pressed into a disk having an inner diameter of 3 cm. The metal amount of this disk was measured using a fluorescent X-ray spectrometer ZSX Primus II manufactured by Rigaku Corporation. Potassium acetate was quantified by determining the amount of potassium atoms and converting it into the amount of potassium acetate.

### <Catalytic Activity Evaluation Test>

A reaction tube (made of SUS316L, inner diameter: 22 mm, length: 480 mm) was packed with 6.7 mL of the catalyst diluted with 75 mL of glass beads. Catalytic activity and selectivity were evaluated by passing a gas having a composition of C₂H₄/O₂/H₂O/HOAc/N₂ = 45/6/5/23/21 (mol%) through the reaction tube at a flow rate of 66.7 NL/h (SV: 10,000 h⁻¹) for 240 hours under conditions of a reaction temperature of 150°C and a reaction pressure of 0.6 MPaG. In general, as the vinyl acetate activity (STY) (g/L-cat·h) increases, the vinyl acetate selectivity decreases. Therefore, the vinyl acetate selectivity at the same vinyl acetate activity was compared among the catalysts. For example, the vinyl acetate selectivity at a vinyl acetate activity of 750 (g/L-cat·h) was calculated by interpolation from a polynomial approximation curve obtained from plots of vinyl acetate activity and vinyl acetate selectivity at each reaction temperature.

Analysis of the reactor outlet gas was performed using the following method.

### 1. Oxygen

Using an absolute calibration curve method, 50 mL of the effluent gas was sampled, and the entire amount was introduced into a 1 mL gas sampler attached to a gas chromatograph, followed by analysis under the following conditions.
Gas chromatography: Gas chromatograph (Shimadzu Corporation
GC-14B) equipped with a gas sampler (MGS-4: measuring tube 1 mL) for Shimadzu gas chromatograph
Column: MS-5A IS 60/80 mesh (3 mmφ × 3 m)
Carrier gas: helium (flow rate: 20 mL/min)
Temperature conditions: detector temperature and vaporization chamber temperature were 110°C, and the column temperature was maintained constant at 70°C
Detector: TCD (He pressure: 70 kPaG, current: 100 mA)

### 2. Acetic acid

Using an internal standard method, 1 mL of 1,4-dioxane was added as an internal standard to 10 mL of the reaction solution to prepare an analysis solution, and 0.2 µL thereof was injected and analyzed under the following conditions.
Gas chromatography: Shimadzu Corporation GC-14B
Column: packed column Thermon 3000 (length: 3 m, inner diameter: 0.3 mm)
Carrier gas: nitrogen (flow rate: 20 mL/min)
Temperature conditions: the detector temperature and vaporization chamber temperature were 180°C; the column temperature was maintained at 50°C for 6 minutes from the start of the analysis, then raised to 150°C at a heating rate of 10°C/min, and maintained at 150°C for 10 minutes
Detector: FID (H₂ pressure: 40 kPaG, air pressure: 100 kPaG)

### 3. Vinyl acetate

Using an internal standard method, 1 g of 1,4-dioxane was added as an internal standard to 6 g of the reaction solution to prepare an analysis solution, and 0.3 µL thereof was injected and analyzed under the following conditions.
Gas chromatography: GC-9A manufactured by Shimadzu Corporation
Column: capillary column TC-WAX (length: 30 m, inner diameter: 0.25 mm, film thickness: 0.5 µm)
Carrier gas: nitrogen (flow rate: 30 mL/min)
Temperature conditions: the detector temperature and vaporization chamber temperature were 200°C; the column temperature was maintained at 45°C for 2 minutes from the start of the analysis, then raised to 130°C at a heating rate of 4°C/min and maintained at 130°C for 15 minutes, followed by raising the temperature to 200°C at a heating rate of 25°C/min and maintaining it at 200°C for 10 minutes.
Detector: FID (H₂ pressure: 60 kPaG, air pressure: 100 kPaG)

The evaluation results of the catalysts are shown in Table 1. The selectivity to vinyl acetate is based on ethylene.

### [Table 1]

**Table 1**

| Examples Comparative Examples | Catalyst | Step 4 | | Step 5 | | Amounts of components per 1kg of carrier [g/kg] | | | | Catalyst metal mass ratio | | | Vinyl acetate selectivity [%]¹⁾ |
|---|---|---|---|---|---|---|---|---|---|---|---|---|---|
| | | Solution B | pH | Reduction temperature [° C] | Reduction time [hr] | Pd | Au | Cu | CH₃COOK | Pd | Au | Cu | |
| Example 1 | Catalyst A | Ion-exchanged water | 7.0 | 5 | 4 | 13.8 | 12.5 | 1.15 | 72.3 | 100.0 | 90.7 | 8.3 | 95.8 |
| Example 2 | Catalyst B | None | - | 5 | 4 | 13.4 | 16.8 | 1.13 | 72.3 | 100.0 | 125.4 | 8.4 | 95.7 |
| Example 3 | Catalyst C | pH buffer solution | 10.0 | 5 | 4 | 12.9 | 12.0 | 1.17 | 72.3 | 100.0 | 93.4 | 9.1 | 95.9 |
| Example 4 | Catalyst D | pH buffer solution | 10.0 | 15 | 4 | 12.9 | 12.0 | 1.17 | 72.3 | 100.0 | 93.4 | 9.1 | 95.9 |
| Comparative Example 1 | Catalyst E | Ion-exchanged water | 7.0 | 25 | 4 | 13.8 | 12.5 | 1.15 | 72.3 | 100.0 | 90.7 | 8.3 | 94.7 |
| Comparative Example 2 | Catalyst F | None | - | 25 | 4 | 13.0 | 8.3 | 1.08 | 72.3 | 100.0 | 63.9 | 8.4 | 94.2 |

| | | | | | | | | | | | | | |
|---|---|---|---|---|---|---|---|---|---|---|---|---|---|
| 1) Selectivity is an interpolated value at a reaction temperature at which the vinyl acetate activity (STY) [g/L-cat-h] is 750 | | | | | | | | | | | | | |

Comparison of Examples 1 to 4 with Comparative Examples 1 and 2 shows that, by lowering the temperature of the solution containing the reducing agent used for reducing the compounds containing palladium, gold, and copper supported on the carrier to metals from 25°C to 15°C or lower, the vinyl acetate selectivity was improved by from 1.0 to 1.7 points, demonstrating a remarkable effect. It is presumed that this effect is due to differences in the alloy state.

## Claims

1. A method for producing a catalyst for vinyl acetate production, the catalyst comprising a carrier, palladium, gold, copper, and an acetate salt, the method comprising:
step 1. impregnating the carrier with an alkaline solution;
step 2. contact-impregnating the carrier with a solution comprising a palladium-containing compound, a gold-containing compound, and a copper-containing compound;
step 5. performing a liquid-phase reduction treatment at a temperature in a range of 0°C to 15°C; and
step 6. supporting an acetate salt on the carrier.

2. The production method according to claim 1, further comprising, between the step 2 and the step 5,
step 3. separating the carrier from the solution in the step 2 when desired supported amounts of the catalyst components have been supported on the carrier.

3. The production method according to claim 2, further comprising, between the step 3 and the step 5,
step 4. bringing the carrier into contact with water or an aqueous solution having a pH of 7.0 to 11.5.

4. The production method according to any one of claims 1 to 3, wherein a mass of supported metallic palladium per 1 kg of the carrier is from 5.00 g to 20.0 g.

5. The production method according to any one of claims 1 to 3, wherein a mass of supported metallic gold per 1 kg of the carrier is from 4.00 g to 20.0 g.

6. The production method according to any one of claims 1 to 3, wherein a mass of supported metallic copper per 1 kg of the carrier is from 0.25 g to 5.00 g.

7. The production method according to any one of claims 1 to 3, wherein a mass of supported acetate salt per 1 kg of the carrier is from 40 g to 125 g.

8. The production method according to any one of claims 1 to 3, wherein a mass ratio of palladium, gold, and copper is 100 : 70 to 130 : 8.3 to 9.5.

9. A method for producing vinyl acetate, wherein a catalyst for vinyl acetate production obtained by the method according to any one of claims 1 to 3 is used, and ethylene, oxygen, and acetic acid are used as raw materials.
